# EUROPÄISCHE PATENTSCHRIFT

(11) **EP 0 922 703 B1**
(45) Veröffentlichungstag und Bekanntmachung des Hinweises auf die Patenterteilung: **04.12.2002**
(21) Anmeldenummer: 98122218.5
(22) Anmeldetag: 23.11.1998
(51) Int. Cl.: C07D 311/58, C07D 313/08, C07D 335/06, C07D 337/08, A61K 31/35, A61K 31/38, A61K 31/55

(54) **Substituierte heterocyclische Benzocycloalkene und ihre Verwendung als analgetisch wirksame Substanzen**
Substituted heterocyclic benzocycloalkenes and their use as analgesically active compounds
Benzocycloalcènes hétérocycliques substitués et leur application comme composés analgésiquement actifs

(30) Priorität: 13.12.1997 DE 19755480
(43) Veröffentlichungstag der Anmeldung: 16.06.1999
(73) Patentinhaber: Grünenthal GmbH, 52078 Aachen (DE)
(72) Erfinder: Zimmer, Oswald Dr., 52146 Würselen (DE); Strassburger, Wolfgang Prof. Dr., 52146 Würselen (DE); Pütz, Claudia Dr., 52349 Düren (DE); Englberger, Werner Dr., 52223 Stolberg (DE); Kögel, Babette-Yvonne Dr., 52379 Langerwehe-Hamich (DE)

(56) Entgegenhaltungen:
- US-A- 3 444 176
- K. SINDELAR ET AL.: COLLECTION OF CZECHOSLOVAK CHEMICAL COMMUNICATIONS, Bd. 33, Nr. 12, 1968, Seiten 4315-27, XP002097420
- M. PROTIVA ET AL.: COLLECTION OF CZECHOSLOVAK CHEMICAL COMMUNICATIONS, Bd. 37, Nr. 3, 1972, Seiten 868-86, XP002097421
- CHEMICAL ABSTRACTS, vol. 74, no. 19, 10. Mai 1971 Columbus, Ohio, US; abstract no. 99909r, Seite 499; XP002097422
- PATENT ABSTRACTS OF JAPAN vol. 10, no. 318 (C-381), 29. Oktober 1986 & JP 61 130286 A (SUNTORY LTD), 18. Juni 1986

## Beschreibung

Die Erfindung betrifft substituierte heterocyclische Benzocycloalkene der allgemeinen Formel I, 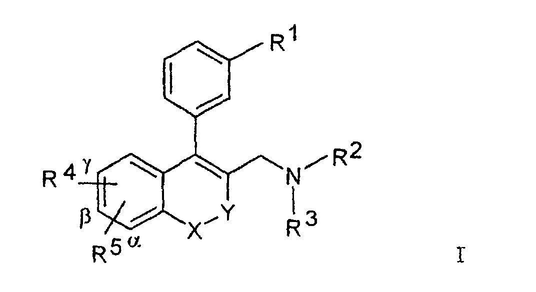 worin
- R¹: OH, C₁₋₆-Alkoxy, -O-(C₃₋₇)-Cycloalkyl;
- R²: C₁₋₆-Alkyl;
- R³: C₁₋₆-Alkyl, -(CH₂)₁₋₂-Aryl, -(CH₂)₁₋₂-Heterocyclyl, -CH₂-CH=C(R⁶)₂, -CH₂- (C₃₋₇)-Cycloalkyl;
- R⁴ und R⁵: gleich oder verschieden voneinander H, Cl, F, CF₃, C₁₋₆-Alkyl, CON(R⁶R⁷), OH, C₁₋₆-Alkoxy, O- (C₃₋₇)-Cycloalkyl, -(CH₂)₀₋₂-Aryl, -O-(CH₂)₀₋₂-Aryl, α,β- oder β,γ -O-(CH₂)₁₋₂-O-, Heterocyclyl, α,β-oder β,γ-Benzo unsubstituiert, mono- oder disubstituiert mit Cl, F, CF₃, OH, C₁₋₆-Alkyl, C₁₋₆-Alkoxy;
- R⁶: H, C₁-C₆-Alkyl;
- R⁷: H, C₁-C₆-Alkyl, -(CH₂)₀₋₂-Aryl, -CH₂-(C₃-C₇)-Cycloalkyl oder
- R⁶ und R⁷: zusammengenommen (-CH₂-)₅₋₇ oder (-CH₂)₂-O-(CH₂)₂-
- X: O, S, SO, SO₂ und
- Y: -(CH₂)₁₋₂-, -CH₂-C(CH₃)₂- oder -C(CH₃)₂-
bedeuten oder pharmazeutisch verwendbare Salze davon, sowie ein Verfahren zur Herstellung und die Verwendung als Arzneimittel.

Verwandte analgetische Verbindungen sind von K. Sindelar et Al., Collection Czechoslov. Chem. Commun. vol. 33 (1968) 4315-4327 und Von M. Protiva et Al., Collection Czechoslov, Chem. Commun. Vol. 37 (1972) 868-886 bekannt.

Klassische Opioide wie das Morphin sind bei der Therapie starker bis stärkster Schmerzen gut wirksam. Ihr Einsatz wird jedoch durch die bekannten Nebenwirkungen, z. B. Atemdepression, Erbrechen, Sedierung und Obstipation und die Toleranzentwicklung eingeschränkt. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs-/Nebenwirkungsprofil als z. B. Morphin verfügen. Während Morphin selektiv an die sogenannten µ-Rezeptoren bindet, wurden die endogenen Enkephaline als δ selektive Peptide charakterisiert. Weitere pharmakologische Untersuchungen haben inzwischen die Existenz mehrerer Subtypen dieser Opioidrezeptoren (µ₁, µ₂, κ₁, κ₂, κ₃, δ₁ und δ₂) wahrscheinlich gemacht.

Kenntnisse über die physiologische Bedeutung von δ-rezeptorselektiven Substanzen sind wesentlich durch die Entdeckung des nicht-peptidischen Antagonisten Naltrindol erweitert worden. So steht inzwischen fest, daß δ-Agonisten über ein eigenständiges antinociceptives Potential verfügen. Neben einer Vielzahl von tierexperimentellen Studien gibt es dazu auch eine Untersuchung mit dem peptidischen Agonisten D-Alanin²-D-Leucin⁵-Enkephalin (DADL) an Krebspatienten, bei denen Morphin keine analgetische Wirkung mehr hatte. Bei intrathekaler Gabe zeigte DADL einen lang anhaltenden analgetischen Effekt.

Deutlich unterscheiden sich δ- von µ-Agonisten in ihrer Wechselwirkung mit dem "endogenen Opioidantagonisten" Cholecystokinin (CCK).

Neben diesem unterschiedlichen Wirkprofil könnte sich auch das Nebenwirkungsprofil der δ-Agonisten von µ-Agonisten unterscheiden, z.B. durch eine geringere Atemdepression.
Potentiell therapeutisch einsetzbar sind diese Verbindungen als Analgetika oder, ganz allgemein, für alle pathologischen Zustände die gewohnlich mit δ-Opiatrezeptoragonisten behandelt werden.

Die der Erfindung zugrundeliegende Aufgabe bestand deshalb darin, analgetisch wirksame Substanzen zu finden, deren biologische Wirksamkeit teilweise oder überwiegend über δ-Opiatrezeptoragonisten vermittelt wird.

Mit den substituierten heterocyclischen Benzocycloalken-Verbindungen der vorliegenden Erfindung sind diese Anforderungen erfüllt worden.

Gegenstand der Erfindung sind substituierte heterocyclische Benzocycloalkene der allgemeinen Formel I, 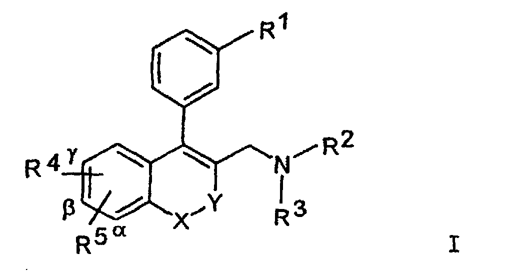 worin
- R¹: OH, C₁₋₆-Alkoxy, -O-(C₃₋₇)-Cycloalkyl;
- R²: C₁₋₆-Alkyl;
- R³: C₁₋₆-Alkyl, -(CH₂)₁₋₂-Aryl, -(CH₂)₁₋₂-Heterocyclyl, -CH₂-CH=C(R⁶)₂, -CH₂-(C₃₋₇)-Cycloalkyl;
- R⁴ und R⁵: gleich oder verschieden voneinander H, Cl, F, CF₃, C₁₋₆-Alkyl, CON(R⁶R⁷), OH, C₁₋₆-Alkoxy, O-(C₃₋₇)-Cycloalkyl, -(CH₂)₀₋₂-Aryl, -O-(CH₂) ₀₋₂-Aryl, α,β- oder β,γ -O-(CH₂)₁₋₂-O-, Heterocyclyl, α,β-oder β,γ-Benzo unsubstituiert, mono- oder disubstituiert mit Cl, F, CF₃, OH, C₁₋₆-Alkyl, C₁₋₆-Alkoxy;
- R⁶: H, C₁-C₆-Alkyl;
- R⁷: H, C₁-C₆-Alkyl,-(CH₂)₀₋₂-Aryl, -CH₂-(C₃-C₇)-Cycloalkyl oder
- R⁶ und R⁷: zusammengenommen (-CH₂-)₅₋₇ oder (-CH₂)₂-O-(CH₂)₂-
- X: O, S, SO, SO₂ und
- Y: -(CH₂)₁₋₂-, -CH₂-C(CH₃)₂- oder -C(CH₃)₂-
bedeuten, oder pharmazeutisch verwendbare Salze davon.

Bevorzugt sind Verbindungen der allgemeinen Formel I, in denen X O, S oder SO und Y -(CH₂)₁₋₂- und R¹ bis R⁷ die Bedeutung gemäß der Definition der allgemeinen Formel I haben oder
X O, Y -(CH₂)₁₋₂- und R¹ bis R⁷ die Bedeutung gemäß der Definition der allgemeinen Formel I haben oder
R⁴ und R⁵ gleich oder verschieden voneinander -O-(CH₂)₀₋₂-Aryl, -(CH₂)₀₋₂-Aryl oder Heterocyclyl und R¹ bis R³, R⁶, R⁷, X und Y die Bedeutung wie oben definiert ist besitzen, oder
R¹ C₁₋₆-Alkoxy oder -O-(C₃₋₇)-Cycloalkyl, R⁴ und R⁵ gleich oder verschieden voneinander -(CH₂)₀₋₂-Aryl oder Heterocyclyl, R², R³, R⁶, R⁷, X und Y die Bedeutung wie oben definiert ist besitzen, oder
R¹ OH oder C₁₋₆-Alkoxy und R² bis R⁷, X und Y die Bedeutung wie weiter oben definiert ist besitzen, oder
Y - (CH₂)₁₋₂-, R² C₁₋₆-Alkyl, X, R¹ und R³ bis R⁷ die Bedeutung gemäß der Definition der allgemeinen Formel I besitzen, oder
Y -(CH₂)₁₋₂-, R¹ OH oder C₁₋₆-Alkoxy, R² und R³ C₁₋₆-Alkyl, X und R⁴ bis R⁷ die Bedeutung gemäß der Definition der allgemeinen Formel I besitzen oder
Y -(CH₂)₁₋₂-, R¹ OH, R² und R³ C₁₋₆-Alkyl, X O und R⁴ bis R⁷ die Bedeutung gemäß der Definition der allgemeinen Formel I besitzen.

Der Ausdruck "C₁₋₆-Alkyl" bedeutet in der vorliegenden Erfindung geradkettige oder verzweigte Kohlenwasserstoffe mit 1-6 Kohlenstoffatomen. Beispielhaft seien Methyl, Ethyl, Propyl, Isopropyl, n-Butyl, sek-Butyl, tert-Butyl, n-Pentyl, Neopentyl und n-Hexyl genannt.

Der Ausdruck "C₁₋₆-Alkoxy" bedeutet im Rahmen der vorliegenden Erfindung geradkettige oder verzweigte Kohlenwasserstoffe mit 1-6 Kohlenstoffatomen wie oben definiert, die über ein Sauerstoffatom gebunden sind.

Der Ausdruck "Aryl" bedeutet im "Rahmen der vorliegenden Erfindung unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituierte Phenyle. Der Ausdruck kann gegebenenfalls auch Naphthyl bedeuten

Unter dem Ausdruck "Heterocyclyl" sind im Rahmen der vorliegenden Erfindung 5- oder 6-gliedrige gesättigte oder ungesättigte, gegebenenfalls mit einem ankondensierten Arylsystem versehene, heterocyclische Verbindungen zu verstehen, die 1 oder 2 Heteroatome aus der Gruppe von Stickstoff, Sauerstoff und/oder Schwefel enthalten.

Beispielhaft seien für die gesättigten Heterocyclyle, 1,4-Dioxan, Tetrahydrofuran, 1,2-Oxathiolan, Pyrrolidin und Piperazin aufgeführt.

Als Beispiel für die Gruppe der ungesattigten Heterocyclyle seien im Rahmen der folgenden Erfindung Furan, Thiophen, Pyrrol, Pyridin, Pyrimidin, 1,3-Thiazol, Oxazol, Isoxazol, Imidazol, Pyrazol, γ-Pyran, γ-Thiapyran, Pyradizin, Pyrazin, 1,4-Thiazin, Chinolin, Isochinolin und Chinazolin aufgeführt.

Unter dem Ausdruck "Silanylverbindung" sind im Rahmen der vorliegenden Erfindung Trialkyl- oder Triarylsilyle, Dialkylarylsilyle oder Diarylalkylsilyle zu verstehen, die als Schutzgruppe für die Hydroxy-Funktion verwendet werden. Beispielhaft seien Triethylsilyl, Tripropylsilyl, Dimethyl-phenylsilyl, Di-tertbutyl-phenylsilyl, Triisopropylsilyl, Dimethylisopropylsilyl, Diethylisopropylsilyl, Dimethylthexyl-silyl, tert-Butyldimethylsilyl, tert-Butyl-diphenylsilyl, Tribenzylsilyl, Tri-p-xylylsilyl, Triphenylsilyl, Diphenyl-methylsilyl oder Propyl-diphenylsilyl erwahnt.

Erfindungsgegenstand ist auch ein Verfahren zur Herstellung der substituierten heterocyclischen Benzocycloalkene der allgemeinen Formel (I), das gekennzeichnet ist durch die Umsetzung eines tertiaren Alkohols der allgemeinen Formel II, 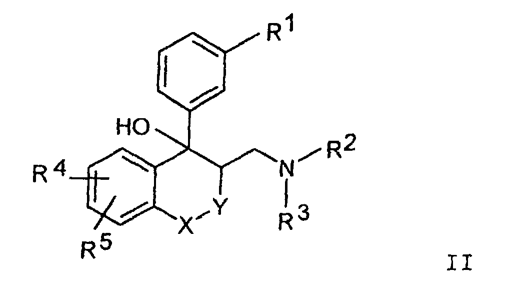 in der R¹ bis R⁷, X und Y dieselbe Bedeutung wie in Formel I haben, mit halbkonzentrierten oder konzentrierten organischen oder anorganischen Säuren, besonders bevorzugt Ameisensäure oder Salzsäure bei Temperaturen zwischen O° C und 100° C, vorzugsweise +50° C, wobei die tertiaren Alkohole der allgemeinen Formel II dadurch erhalten werden, daß Mannich-Basen der allgemeinen Formel III 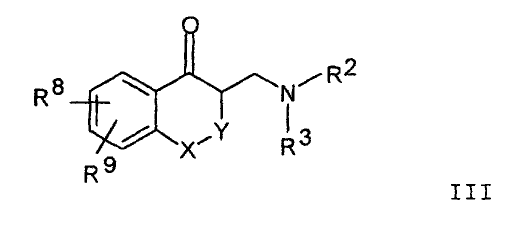 wobei R², R³, X und Y dieselbe Bedeutung wie in Formel I besitzen und R⁸ wie R⁴ und R⁹ wie R⁵ definiert sind, mit der Ausnahme, daß eine gegebenenfalls vorhandene Hydroxy-Funktion in geschützter Form als Benzyloxy- oder Silanyloxygruppe vorliegt, mit einer metallorganischen Verbindung der Formel IV 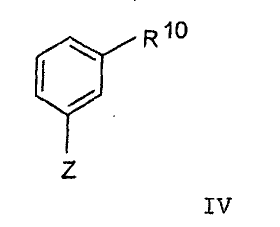 in der Z für MgCl, MgBr, MgI oder Li steht und R¹⁰ die Bedeutung gemäß R¹ besitzt, mit der Ausnahme, daß eine gegebenenfalls vorhandene Hydroxy-Funktion in geschützter Form als Benzyloxy- oder Silanyloxy-verbindung z. B. als tert-Butyl-diphenylsilyloxy vorliegt, die zu einer Verbindung der Formel IIa umgesetzt werden 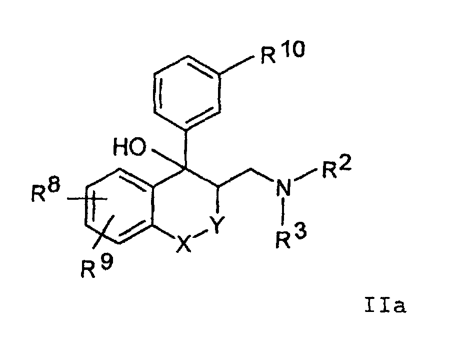 und diese dann in Verbindungen der Formel II übergeführt werden.

Die Umsetzung der Verbindungen III und IV wird in einem aliphatischen Ether, beispielsweise Diethylether und/oder Tetrahydrofuran, bei Temperaturen zwischen -70° C und +60° C durchgeführt. Verbindungen der Formel IV, in denen Z für ein Lithiumatom steht, werden dabei aus Verbindungen der Formel IV, in denen Z Br oder I bedeutet, durch Halogen-Lithiumaustausch mittels z. B. einer n-Butyllithium/n-Hexan-Losung gewonnen.

Für die Umsetzung einer Verbindung der Formel IIa zu einer der Formel II stehen in Abhängigkeit von R⁸, R⁹ bzw. R¹⁰ mehrere Methoden zur Verfügung.

Stehen R⁸, R⁹ und/oder R¹⁰ für eine Benzyloxygruppe, so geschieht dies zweckmäßig durch eine reduktive Debenzylierung mit katalytisch aktiviertem Wasserstoff, wobei Platin oder Palladium, absorbiert auf einem Trägermaterial wie Aktivkohle, als Katalysator dienen. Die Reaktion wird in einem Lösungsmittel wie Essigsäure oder einem C₁₋₄-Alkylalkohol bei Drücken von 1 bis 100 bar und Temperaturen von +20° C bis +100° C durchgeführt, wobei die Verbindung IIa vorzugsweise in Form eines ihrer Salze eingesetzt wird.

Stellen R⁹ und/oder R¹⁰ eine Silanyloxygruppe dar, so erfolgt die Abspaltung der Schutzgruppe dadurch, daß man die entsprechende Verbindung der Formel IIa bei +20° C in einem inerten Lösungsmittel wie Tetrahydrofuran, Dioxan oder Diethylether mit Tetra-n-butylammoniumfluorid umsetzt oder mit einer methanolischen Lösung von Chlorwasserstoff behandelt.

Stehen R⁸, R⁹ und/oder R¹⁰ in der Verbindung der Formel IIa für den Methoxyrest, läßt sich durch Umsetzung mit Diisobutylaluminiumhydrid in einem aromatischen Kohlenwasserstoff wie Toluol bei einer Temperatur zwischen 60° C und 130° C die Verbindung der Formel II herstellen, in der R¹ für eine Hydroxygruppe steht. Man kann auch direkt die analoge Verbindung der Formel I erhalten, indem man IIa entweder mit einer Lösung von Bromwasserstoff in Eisessig oder konzentrierter Bromwasserstoffsäure zum Rückfluß erhitzt.

Auch aus Verbindungen der Formel I, in denen R¹ und/oder R⁴ bzw. R⁵ für eine Methoxygruppe stehen, lassen sich wie oben beschrieben durch Umsetzung mit Diisobutylaluminiumhydrid Verbindungen der Formel I mit R¹ und/oder R² bzw. R⁵ gleich OH erhalten. Dies ist auch durch Umsetzung mit Methansulfonsäure/Methionin bei Temperaturen zwischen 20° C und 50° C möglich.

Aus Verbindungen der Formel I, in denen X für ein S-Atom steht, lassen sich Verbindungen der Formel I in denen X die SO- oder SO₂-Gruppe bedeutet, durch Oxidation mit Wasserstoffperoxid (30 Gew.% in Wasser) und Essigsäure als Lösungsmittel bei Temperaturen zwischen +20° C und +60° C herstellen.

Die Verbindungen der Formel I lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diethylether, Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

### Beispiele

Die folgenden Beispiele dienen zur näheren Erläuterung der vorliegenden Erfindung.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Der Ausdruck TRIS-HCl bedeutet Tris-(hydroxymethyl)-aminomethan-hydrochlorid.
(w/v) Gewicht/Volumen

### Beispiel 1

### 3-(4-Dimethylaminomethyl-2,3-dihydro-benzo[b]thiepin-5-yl)-phenol, Hydrochlorid

### 1. Stufe

### (RS)-4-Dimethylaminomethyl-3,4-dihydro-2H-benzo[b]thiepin-5-on, Hydrochlorid

Eine Losung von 32,1 g 3,4-Dihydro-2H-benzo[b]thiepin-5-on in 320 ml Actonitril wurde mit 16,9 g N,N-Dimethylmethylenimmoniumchlorid und drei Tropfen Acetylchlorid versetzt und das Gemisch 72 Stunden bei 20° C geruhrt. Dann wurde mit 100 ml Diethylether verdünnt, das kristalline Produkt isoliert, mit Diethylether gewaschen und im Vakuum bei 40° C getrocknet. Man erhielt so 44,1 g (90,0 % d. Th.) der Titelverbindung in Form weißer Kristalle.
Schmelzpunkt: 183 - 185° C

### 2. Stufe:

### (4RS, 5RS)-5-[3-Tert-butyl-diphenyl-silanyloxy)-phenyl]-4-dimethylaminomethyl-2,3,4,5-tetrahydrobenzo[b]thiepin-5-ol

Eine Lösung von 32,9 g (3-Bromphenoxy)-tert-butyldiphenyl-silan in 250 ml trockenem Tetrahydrofuran wurde bei -40° C unter Rühren und Überleiten von trockenem Stickstoff tropfenweise mit 50 ml einer 1,6-molaren Lösung von n-Butyl-lithium in n-Hexan versetzt. Nach beendeter Zugabe wurde noch 30 Minuten bei -40° C bis -30° C gerührt, dann tropfte man bei derselben Temperatur eine Lösung von 15,1 g der freien Base des Produktes aus Stufe 1 in 40 ml trockenem Tetrahydrofuran hinzu. Es wurde weitere 4 Stunden bei dieser Temperatur gerührt, dann durch Zugabe von 50 ml einer gesättigten Ammoniumchloridlösung zersetzt. Die organische Phase wurde abgetrennt, die wässrige noch zweimal mit Essigsäureethylester extrahiert. Die vereinigten organischen Phase wurden mit einer gesättigten Natriumchloridlösung gewaschen und über Natriumsulfat getrocknet. Nach Abfiltrieren und Abdampfen der Lösungsmittel im Vakuum blieben 11,4 g (31,3 % d. Th.) der Titelverbindung in Form eines farblosen viskosen Öls zuruck.

### 3. Stufe:

### (4RS, 5RS)-4-Dimethylaminomethyl-5-(3-hydroxy-phenyl)-2,3,4,5-tetrahydro-benzo[b]thiepin-5-ol

Eine Lösung von 11,4 g des Produktes aus Stufe 2 in 200 ml trockenem Tetrahydrofuran wurde bei 5° C unter Rühren tropfenweise mit 22 ml einer 1 M Lösung von Tetra-n-butylammoniumfluorid in Tetrahydrofuran versetzt. Nach beendeter Zugabe rührte man 3 Stunden bei 20° C, versetzte mit 50 ml einer gesättigten Lösung von Ammoniumchlorid und extrahierte dreimal mit je 50 ml Essigsäureethylester. Die Extrakte wurden mit gesättigter Natriumchloridlösung gewaschen, über Natriumsulfat getrocknet und im Vakuum eingedampft. Man reinigte den Rückstand säulenchromatographisch mit Essigsäureethylester/Methanol = 9/1 als Elutionsmittel und erhielt so 6 g (90,8 % d. Th.) der Titelverbindung in Form weißer Kristalle, die bei 188 - 190° C schmolzen.

### 4. Stufe:

### 3-(4-Dimethylaminomethyl-(2,3-dihydro-benzo[b]thiepin-5-yl)-phenol, Hydrochlorid

Eine Lösung von 4,95 g der Verbindung aus Stufe 3 in 50 ml Tetrahydrofuran wurde mit 150 ml 6N Salzsäure versetzt und das Gemisch 48 Stunden bei 20° C, dann 24 Stunden bei 60° C gerührt. Man alkalisierte mit Natronlauge und extrahierte dreimal mit je 100 ml Essigsäureethylester. Die Extrakte wurden mit gesättigter Natriumchloridlösung gewaschen, über Natriumsulfat getrocknet und im Vakuum eingedampft. Dabei blieben 4,44 g (95,0 % d. Th.) der freien Base der Titelverbindung als weißer Feststoff zurück (Schmelzpunkt: 217 - 219° C), die mit Trimethylchlorsilan/Wasser in einem Lösungsmittelgemisch aus 2-Butanon und Tetrahydrofuran (1/2) ins Hydrochlorid übergeführt wurden.
Schmelzpunkt: 251 - 253° C

### Beispiel 2

Unter Einsatz entsprechender cyclischer Ketone anstelle von 3,4-Dihydro-2H-benzo[b]thiepin-5-on in Stufe 1 wurden unter Anwendung der in Beispiel 1 beschriebenen Verfahrensweise analog erhalten:
- 2a:: 3-(8-Chlor-3-dimethylaminomethyl-2H-chromen-4-yl)-phenol, Hydrochlorid
- 2b:: 3-(3-Dimethylaminomethyl-2H-benzo[g]chromen-4-yl-phenol, Hydrochlorid
Schmelzpunkt: 232 - 235° C
- 2c:: 3-(2-Dimethylaminomethyl-3H-4-thiaphenanthren-1-yl)-phenol, Hydrochlorid
Schmelzpunkt: 246 - 248,5° C
- 2d:: 3- (7-Dimethylaminomethyl-2, 3-dihydro-6H-1, 4, 5, - trioxa-phenanthren-8-yl)-phenol, Hydrochlorid
Schmelzpunkt: 229 - 231° C
- 2e:: 3-(4-Dimethylaminomethyl-2,3-dihydrobenzo[b]oxepin-5-yl)-phenol, Hydrochlorid
Schmelzpunkt: 235 - 237° C
- 2f:: 3-(3-Dimethylaminomethyl-6-methoxy-2,2-dimethyl-2H-chromen-4-yl)-phenol, Hydrochlorid
Schmelzpunkt: 217 - 219° C
- 2g:: 3-(4-Dimethylaminomethyl-8-methoxy-2,3-dihydro-benzo[b]oxepin-5-yl)-phenol, Hydrochlorid
Schmelzpunkt: 206 - 208° C
- 2h:: 3-(4-Dimethylaminomethyl-8-methoxy-2,3-dihydro-benzo[b]thiepin-5-yl)-phenol, Hydrochlorid
Schmelzpunkt: 232 - 235° C
- 2i:: 3-(4-Dimethylaminomethyl-8-fluor-2,3-dihydro-benzo[b]thiepin-5-yl)-phenol, Hydrochlorid
Schmelzpunkt: Zersetzung ab 130° C
- 2j:: 3-(4-Dimethylaminomethyl-8-fluor-2,3-dihydro-benzo[b]oxepin-5-yl)-phenol, Hydrochlorid
Schmelzpunkt: 245 - 247° C
- 2k:: 3-(7-tert-Butyl-4-dimethylaminomethyl-2,3-dihydro-benzo[b]oxepin-5-yl)-phenol, Hydrochlorid
Schmelzpunkt: 264 - 266° C
- 21:: 3-(7,8-Dichlor-4-dimethylaminomethyl-2,3-dihydro-benzo[b]oxepin-5-yl)-phenol, Hydrochlorid
Schmelzpunkt: 219 - 220° C
- 2m:: 3-(4-Dimethylaminomethyl-9-methoxy-2,3-dihydro-benzo[b]oxepin-5-yl)-phenol, Hydrochlorid
Schmelzpunkt: 194 - 196° C
- 2n:: 3- (8-Benzyloxy-4 -dimethylaminomethyl-2,3-dihydro-benzo[b]oxepin-5-yl)-phenol, Hydrochlorid
Schmelzpunkt: 234 - 236° C

### Beispiel 3

### 3-(4-Dimethylaminomethyl-1-oxo-2,3-dihydro-1H-1λ⁴-benzo[b]thiepin-5-yl)-phenol, Hydrochlorid (Racemat und Enantiomere)

Ein Gemisch aus 1,74 g des Produktes aus Beispiel 1, 17 ml Eisessig und 1,6 ml einer wässrigen Lösung von Wasserstoffperoxid (30 Gew.-% H₂O₂) wurde 2 Stunden bei 20° C gerührt. Danach verdünnte man mit 50 ml Wasser und alkalisierte mit Natronlauge bis zu einem pH-Wert von 9. Es wurde dreimal mit je 30 ml Essigsäureethylester extrah iert. Die vereinigten Extrakte wurden mit gesättigter Natriumchloridlösung gewaschen, über Natriumsulfat getrocknet und im Vakuum eingedampft. Der Rückstand wurde wie in Beispiel 1, Stufe 4, beschrieben ins Hydrochlorid übergeführt. Man erhielt so 1,68 g (92,4 % d. Th.) der racemischen Titelverbindung in Form weißer Kristalle, die bei 208 - 210° C schmolzen. Durch HPLC an einer stationären Phase mit n-Hexan/IsopropanoI/Diethylamin = 950/50/1 ließen sich daraus die Enantiomeren in reiner Form gewinnen.

### Beispiel 4

### 3-(4-Dimethylaminomethyl-1,1-dioxo-2,3-dihydro-1H-1λ⁶benzo[b]thiepin-5-yl)-phenol, Hydrochlorid

0,91 g des Produktes aus Beispiel 3 in 4,5 ml Eisessig wurden mit 0,5 ml einer wassrigen Losung von Wasserstoffperoxid (30 Gew.-% H₂O₂) 24 Stunden bei 45° C gerührt. Nach Aufarbeitung wie in Beispiel 2 beschrieben, säulenchromatographischer Reinigung des Rohproduktes mit Essigsäureethylester/Methanol = 5/1 als Elutionsmittel und Überführen der gereinigten Substanz ins Hydrochlorid erhielt man 0,67 g (70,3 % d. Th.) der Titelverbindung in Form weißer Kristalle, die bei 263 - 266° C schmolzen.

### Beispiel 5

### 3-(3-Dimethylaminomethyl-2H-thiochromen-4-yl)-phenol, Hydrochlorid

### 1. Stufe:

### (3RS, 4RS)-3-Dimethylaminomethyl-4-(3-methoxy-phenyl)-thiochroman-4-ol

Aus 0,73 g Magnesiumspänen und 5,61 g 1-Brom-3-methoxybenzol in 20 ml trockenem Tetrahydrofuran wurde unter leichtem Sieden das entsprechende Grignardreagenz dargestellt. Dazu tropfte man bei +5 bis 10° C eine Lösung von 4,43 g (RS)-3-Dimethylaminomethyl-thiochroman-4-on in 10 ml trockenem Tetrahydrofuran. Es wurde 6 Stunden bei 20° C nachgerührt, dann mit 10 ml einer gesättigten Ammoniumchloridlösung zersetzt. Man extrahierte dreimal mit Diethylether, wusch die vereinigten Extrakte mit einer gesättigten Natriumchloridlösung und trocknete über Natriumsulfat. Das nach Abdampfen der flüchtigen Bestandteile zurückbleibende Rohprodukt wurde saulenchromatographisch mit Essigsäureethylester als Elutionsmittel gereinigt, wobei 3,68 g (55,8 d. Th.) der Titelverbindung anfielen.

### 2. Stufe:

### 3-(3-Dimethylaminomethyl-2H-thiochromen-4-yl)-phenol, Hydrochlorid

3,3 g des Produkts aus Stufe 1 wurden mit 90 ml einer Lösung von Bromwasserstoff in Eisessig (33 % HBr) 8 Stunden bei 100° C bis 110° C gerührt. Dann wurde im Vakuum eingedampft und der Rückstand in 100 ml Wasser aufgenommen. Es wurde mit Natriumcarbonat alkalisiert und dreimal mit je 30 ml Dichlormethan extrahiert. Man trocknete die Extrakte über Natriumsulfat, dampfte ein und reinigte den Rückstand säulenchromatographisch mit Essigsäureethylester als Elutionsmittel. Die so erhaltene Base der Titelverbindung wurde mit Trimethylchlorsilan/Wasser in 2-Butanon ins Hydrochlorid übergeführt.
Ausbeute: 1,40 g (41,7 % d. Th.)
Schmelzpunkt: 203 - 208° C

### Beispiel 6

### [4-(3-Methoxy-phenyl)-2H-chromen-3-ylmethyl]-dimethylamin

### 1. Stufe:

### (3RS, 4RS)-3-Dimethylaminomethyl-4-(3-methoxy-phenyl)-chroman-4-ol

Bei Umsetzung von 4,11 g (RS)-3-Dimethylaminomethylchroman-4-on mit dem Grignardreagenz aus 0,73 g Magnesiumspänen und 5,61 g 1-Brom-3-methoxybenzol nach der in Beispiel 5, Stufe 1, beschriebenen Verfahrensweise und analoger Reinigung erhielt man 3,91 g (62,4 % d. Th.) der Titelverbindung.

### 2. Stufe:

### [4-(3-Methoxy-phenyl)-2H-chromen-3-ylmethyl]-dimethylamin

Eine Lösung von 3,9 g des Produkts aus Stufe 1 in 20 ml Ethanol wurde wie in Beispiel 1, Stufe 4, beschrieben mit 14,5 ml 6N Salzsäure für 2 Stunden bei 20° C zur Reaktion gebracht. Nach analoger Aufarbeitung und säulenchromatographischer Reinigung mit Essigsaureethylester ab Elutionsmittel erhielt man 2,1 g (57,1 % d. Th.) der Titelverbindung als nahezu farbloses Öl, das bei 4° C erstarrt (Schmelzpunkt: 68 - 71° C)

### Beispiel 7

### 3-(3-Dimethylaminomethyl-2H-chromen-4-yl)-phenol, Hydrochlorid

Eine Lösung von 0,59 g des Produkts aus Beispiel 6 in 10 ml Methansulfonsäure wurde unter N₂-Atmosphäre mit 0,59 g Methionin versetzt, wobei eine braune Lösung entstand. Diese wurde eine Stunde bei 20° C gerührt und dann auf Eis/Wasser geschüttet. Man setzte 50 ml Essigsäureethylester hinzu und alkalisierte mit einer wässrigen Natriumcarbonatlösung. Die organische Phase wurde abgetrennt, die wässrige noch zweimal mit je 20 ml Essigsäureethylester extrahiert. Die organischen Phasen wurden mit gesättigter Natriumchloridlösung gewaschen, über Natriumsulfat getrocknet und im Vakuum von den flüchtigen Bestandteilen befreit. Den Rückstand reinigte man säulenchromatographisch mit Essigsäureethylester/Methanol = 5/1. Das so erhaltene Produkt wurde wie in Beispiel 1, Stufe 4, beschrieben ins Hydrochlorid übergeführt.
Ausbeute: 0,26 g (48,1 d. Th.)
Schmelzpunkt: 213 - 215° C

### Beispiel 8

### 3-Dimethylaminomethyl-4-(3-hydroxy-phenyl)-2,2-dimethyl-2H-chromen-6-ol, Hydrochlorid

### 1. Stufe:

### (3RS,4RS)-6-Benyloxy-3-dimethylaminomethyl-4-(3-hydroxy-phenyl)-2,2-dimethyl-chroman-4-ol, Hydrochlorid

Unter Anwendung der in Beispiel 1, Stufen 1-3, beschriebenen Reaktionsfolge und der dort eingesetzten Reagenzien, jedoch unter Verwendung von 6-Benzyloxy-2,2-dimethylchroman-4-on anstelle von 3,4-Dihydro-2Hbenzo[b]thiepin-5-on, ließ sich die freie Base der Titelverbindung erhalten, die wie in Beispiel 1, Stufe 4, beschrieben ins Hydrochlorid übergeführt wurde.
Schmelzpunkt: 142 - 143° C

### 2. Stufe:

### (3RS, 4RS)-3-Dimethylaminomethyl-4-(3-hydroxy-phenyl)-2, 2-dimethyl-chroman-4, 6-diol, Hydrochlorid

1,08 g des Produkts aus Stufe 1, gelöst in 15 ml wasserfreiem Methanol, wurden über 0,11 g Palladium auf Aktivkohle (10 % Pd) katalytisch hydriert. Nach Abfiltrieren des Katalysators und Abdampfen des Lösungsmittels im Vakuum blieben 0,83 g der Titelverbindung zurück, die für die weitere Umsetzung genügend rein waren.

### 3. Stufe:

### 3-Dimethylaminomethyl-4-(3-hydroxy-phenyl)-2,2-dimethyl-2H-chromen-6-ol, Hydrochlorid

0,83 g des Produkts aus Stufe 2 wurden mit 20 ml 6N Salzsäure versetzt und die resultierende Losung 2 Stunden bei 20° C gerührt. Dann alkalisierte man mit verdünnter Natronlauge (pH 8 - 9) und extrahierte dreimal mit je 20 ml Dichlormethan. Die vereinigten Extrakte wurden über Natriumsulfat getrocknet und im Vakuum eingedampft. Der Rückstand wurde säulenchromatographisch mit Essigsäureethylester/Methanol = 5/1 als Eulutionsmittel gereinigt. Nach Überführen ins Hydrochlorid mit Chlortrimethylsilan/Wasser in 2-Butanon fielen 0,57 g (72,2 % d. Th.) der Titelverbindung in Form weißer Kristalle an, die bei 195 - 198° C schmolzen.

### Beispiel 9

Unter Einsatz der Produkte aus den Beispielen 2h und 2i anstelle des Produkts aus Beispiel 1 wurden bei Anwendung der in Beispiel 3 beschriebenen Verfahrensweise analog erhalten:
- 9a:: 3-(4-Dimethylaminomethyl-8-methoxy-1-oxo-2,3-dihydro-1H-1λ4-benzo[b]thiepin-5-yl)-phenol, Hydrochlorid
Schmelzpunkt: ab 222° C unter Zersetzung
- 9b:: 3-(4-Dimethylaminomethyl-8-fluor-1-oxo-2,3-dihydro-1H-1λ4-benzo[b]thiepin-5-yl)-phenol, Hydrochlorid
Schmelzpunkt: ab 198° C unter Zersetzung

### Beispiel 10

Unter Einsatz des Produkts aus Beispiel 9a anstelle des Produkts aus Beispiel 3 wurde bei Anwendung der in Beispiel 4 beschriebenen Verfahrensweise analog erhalten:
3-(4-Dimethylaminomethyl-8-methoxy-1,1-dioxo-2,3-dihydro-1H-1λ6-benzo[b]thiepin-5-yl)-phenol, Hydrochlorid
Schmelzpunkt: 253 - 256° C

### Beispiel 11

Unter Anwendung der in Beispiel 7 beschriebenen Verfahrensweise, jedoch unter Einsatz der Produkte aus Beispiel 2g bzw. 2h anstelle des Produkts aus Beispiel 6, wurden analog erhalten:
- 11a:: 4-Dimethylaminomethyl-5-(3-hydroxy-phenyl)-2, 3-dihydro-benzo[b]oxepin-8-ol, Hydrochlorid
Schmelzpunkt: ab 103° C unter Zersetzung
- 11b:: 4-Dimethylaminomethyl-5-(3-hydroxy-phenyl)-2,3-dihydro-benzo[b]thiepin-8-ol, Hydrochlorid
Schmelzpunkt: ab 117° C unter Zersetzung

### Beispiel 12

### 4-Dimethylaminomethyl-5-(3-hydroxy-phenyl)-2,3-dihydrobenzo[b]oxepin-7-carbonsaurediethylamid, Hydrochlorid

### 1. Stufe: 7-Brom-3,4-dihydro-2H-benzo[b]oxepin-5-on, Ethylenacetal

Ein Gemisch aus 24,1 g 7-Brom-3,4-dihydro-2H-benzo [b]oxepin-5-on, 8,5 ml Ethylenglykol und 0,35 g p-Toluolsulfonsaure Monohydrat wurde am Wasserabscheider 24 Stunden zum Ruckfluß erhitzt. Nach Abtrennen des Reaktionswassers gab man 4 g pulverisiertes Kaliumcarbonat zum Reaktionsgemisch und ruhrte eine Stunde bei 20° C. Danach wurde vom Salz abfiltriert und das Filtrat im Vakuum eingedampft. Dabei blieben 27 g der Titelverbindung als braunes Öl zurück.

### 2. Stufe: 5-Oxo-2,3,4,5-tetrahydro-benzo[b]oxepin-7-carbonsäure, Ethylenacetal

Eine Lösung von 27 g des Produkts aus Stufe 1 in 280 ml wasserfreiem Tetrahydrofuran wurde bei -50° C unter Rühren und Überleiten von Stickstoff tropfenweise mit 62 ml einer 1,6 molaren Lösung von n-Butyllithium in n-Hexan versetzt. Nach beendeter Zugabe rührte man noch 30 Minuten weiter und leitete dann bei -40 bis -50° C bis zur Sättigung Kohlendioxid in die Lösung ein. Man ließ innerhalb von 3 Stunden auf 20° C erwärmen, zersetzte durch Zugabe von 50 ml einer gesattigten Ammoniumchloridlösung, trennte die organische Phase ab und extrahierte die wässrige noch zweimal mit je 50 ml Essigsäureethylester. Die vereinigten organischen Phasen wurden mit einer gesättigten Natriumchloridlösung gewaschen und über Natriumsulfat getrocknet. Den durch Eindampfen im Vakuum erhaltenen Rückstand reinigte man säulenchromatographisch mit Essigsäureethylester als Elutionsmittel und erhielt so 16,4 g (69,1 % d. Th.) der Titelverbindung in Form eines Öls.

### 3. Stufe: 5-Oxo-2,3,4,5-tetrahydro-benzo[b]oxepin-7-carbonsaurediethylamid, Ethylenacetal

Eine Lösung von 16,1 g des Produkts aus Stufe 2 in 40 ml Cyclohexan und 25 ml Thionylchlorid wurde unter einer N₂-Atmosphare 2 Stunden bei 20° C gerührt. Dann wurden die fluchtigen Bestandteile im Vakuum sorgfältig abgedampft. Das zurückbleibende rohe Carbonsaurechlorid löste man in 70 ml Tetrahydrofuran und tropfte die Losung unter Fuhren und Eiswasserkühlung zu einer Losung von 8,7 ml Diethylamin in 150 ml Tetrahydrofuran. Danach wurde noch 2 Stunden bei 20° C gerührt. Es wurde vom Feststoff abgesaugt, dieser gründlich mit Tetrahydrofuran gewaschen und das Filtrat im Vakuum eingedampft. Der Rückstand wurde säulenchromatographisch mit Essigsäureethylester/n-Hexan = 3/1 als Elutionsmittel gereinigt, wobei 8,9 g (45,5 % d. Th.) der Titelverbindung als Öl anfielen.

### 4. Stufe: 5-Oxo-2,3,4,5-tetrahydro-benzo[b]oxepin-7-carbonsäurediethylamid

Eine Mischung aus 8,7 g des Produkts aus Stufe 3, 15 ml Tetrahydrofuran und 30 ml 1N Salzsäure wurde 20 Stunden bei 20° C gerührt. Dann wurde dreimal mit Essigsäureethylester extrahiert. Man wusch die Extrakte mit einer gesättigten Natriumchloridlösung, trocknete über Natriumsulfat und dampfte im Vakuum ein. Dabei blieben 7,37 g (98,9 % d. Th.) der Titelverbindung als leicht gelbes, viskoses Öl zurück.

### 5. Stufe: (RS)-4-Dimethylaminomethyl-5-oxo-2,3,4,5-tetrahydro-benzo[b]oxepin-7-carbonsäurediethylamid, Hydrochlorid

7,2 g des Produkts aus Stufe 4 wurden wie in Beispiel 1, Stufe 1, beschrieben mit 2,7 g N,N-Dimethylmethylenimmoniumchlorid zur Reaktion gebracht. Man erhielt so 8,8 g (90,1 % d. Th.) der Titelverbindung in Form weißer Kristalle, die bei 178 - 180° C schmolzen.

### 6. Stufe: (4RS, 5RS)-5-[3-(tert-Butyl-dimethyl)-silanyloxy)-phenyl]-4-dimethylaminomethyl-5-hydroxy-2, 3, 4, 5-tetrahydro-benzo[b]oxepin-7-carbonsaurediethylamid

9,5 g (3-Bromphenoxy)-tert-butyl-diphenyl-silan, 20,2 ml einer 1,6 molaren Lösung von n-Butyllithium in n-Hexan und 7,8 g des Produkts aus Stufe 5 als freie Base wurden nach der in Beispiel 1, Stufe 2, beschriebenen Verfahrensweise zur Reaktion gebracht. Nach analoger Aufarbeitung und säulenchromatographischer Reinigung mit Essigsäureethylester/Methanol = 5/1 als Elutionsmittel erhielt man 8,29 g (64,3 % d. Th.) der Titelverbindung als leicht gelbes Öl.

### 7. Stufe: (4RS, 5RS)-4-Dimethylaminomethyl-5-hydroxy-5-(3-hydroxy-phenyl)-2,3,4,5-tetrahydro-benzo[b] oxepin-7-carbonsäurediethylamid

8,2 g des Produkts aus Stufe 6 und 50 ml 6N Salzsäure wurden 48 Stunden bei 20° C gerührt. Nach Alkalisieren mit Natronlauge wurde dreimal mit je 30 ml Essigsäureethylester extrahiert. Die Extrakte wurden mit gesättigter Natriumchloridlösung gewaschen, über Natriumsulfat getrocknet und im Vakuum eingedampft. Durch säulenchromatographische Reinigung des Rückstands mit Essigsäureethylester/Methanol = 3/1 als Elutionsmittel erhielt man 5,32 g (82,8 % d. Th.) der Titelverbindung.

### 8. Stufe: 4-Dimethylaminomethyl-5-(3-hydroxy-phenyl)-2,3-dihydro-benzo[b]oxepin-7-carbonsäurediethylamid, Hydrochlorid

5,6 g des Produkts aus Stufe 7 wurden in Form des Hydrochlorids mit 70 ml Ameisensäure unter Rühren für 2 Stunden auf 110° C Badtemperatur erhitzt. Nach dem Abkuhlen wurde mit Natronlauge und Kaliumcarbonat alkalisiert (pH 9) und dreimal mit Dichlormethan extrahiert. Die Extrakte wurden mit gesättigter Natriumchloridlosung gewaschen, über Natriumsulfat getrocknet und im Vakuum eingedampft. Aus dem Ruckstand stellte man mit Trimethylchlorsilan/Wasser in 2-Butanon das Hydrochlorid dar. Es fielen dabei 4,91 g (91,3 % d. Th.) der Titelverbindung in Form weißer Kristalle an, die ab 234° C unter Zersetzung schmolzen.

### Beispiel 13

### 3-{4-[(Methyl-phenethyl-amino)-methyl]-2,3-dihydrobenzo[b]oxepin-5-yl}-phenol, Hydrochlorid

### 1. Stufe: (RS)-4-[(Methyl-phenethyl-amino)-methyl]-3,4-dihydro-2H-benzo[b]oxepin-5-on

Ein Gemisch aus 24,4 g 3,4-Dihydro-2H-benzo[b]oxepin-5-on, 17,2 g N-Methyl-2-phenylethylamin, Hydrochlorid und 3,0 g Paraformaldehyd in 200 ml Eisessig wurde 3 Stunden auf 100° C erhitzt. Danach wurde im Vakuum das Lösungsmittel abgedampft und der Rückstand in 200 ml Wasser aufgenommen. Man extrahierte dreimal mit je 100 ml Diethylether. Die wäßrige Phase wurde mit Kaliumcarbonat alkalisiert und dreimal mit je 100 ml Dichlormethan extrahiert. Nach Waschen der Extrakte mit gesättigter Natriumchloridlösung, Trocknen über Natriumsulfat und Eindampfen im Vakuum blieben 23,9 g (77,2 % d. Th.) der Titelverbindung als gelbliches Ol zurück.

### Endstufe: 3-{4-[(Methyl-phenethyl-amino)-methyl]-2,3-dihydro-benzo[b]oxepin-5-yl}-phenol, Hydrochlorid

Das Produkt aus Stufe 1 wurde nach der in Beispiel 1, Stufen 2 - 4, beschriebenen Verfahrensweise weiter umgesetzt. Man erhielt so die Titelverbindung in Form weißer Kristalle, die ab 135° C unter Zersetzung schmolzen.

### Beispiel 14

### 3-{4-[(Cyclopropyl-methyl-amino)-methyl]-2,3-dihydrobenzo[b]oxepin-5-yl}-phenol, Hydrochlorid

Unter Verwendung von N-(Cyclopropylmethyl)-methylamin, Hydrochlorid in Beispiel 13, Stufe 1, und weiterer Umsetzung des so erhaltenen Produkts nach der in Beispiel 1, Stufen 2 - 4, beschriebenen Verfahrensweise wurde analog die Titelverbindung in Form weißer Kristalle erhalten, die bei 216 - 218° C schmolzen.

### δ-Opiatrezeptor-Bindungsuntersuchungen

Die Untersuchungen zur Bestimmung der Affinität der erfindungsgemäßen Verbindungen der Formel I zum δ-Opiatrezeptor, die als Voraussetzung für analgetische Eigenschaften entscheidend ist, wurde an Hirnmembranhomogenaten (Homogenat von Rattenhirn ohne Cerebellum, Pons und Medulla oblongata von männlichen Wistar-Ratten) durchgeführt. Zum Nachweis der δ-Opiatrezeptoreigenschaften verwendete man ausgewählte Verbindungen der Formel I.

Hierzu wurde das jeweils frisch präparierte Rattenhirn unter Eiskühlung in 50 mmol/l Tris-HCl (pH 7,4) homogenisiert und für 10 Minuten bei 5.000 g und 4°C zentrifugiert. Nach Dekantieren und Verwerfen des Uberstandes, erneutem Aufnehmen und Homogenisieren des Membransedimentes in 50 mmol/l Tris-HCl (pH 7,4) wurde das Homogenat anschließend für 20 Minuten bei 20.000 g und 4°C zentrifugiert. Dieser Waschschritt wurde nochmals wiederholt. Danach wurde der Überstand dekantiert und das Membransediment in kaltem 50 mmol/l Tris-HCl , 20 Glycerin (w/v), 0,01 Bacitracin (w/v) (pH 7,4) homogenisiert und in Aliquoten bis zur Testung eingefroren.

Für die Rezeptorbindungstestung wurden die Aliquote aufgetaut und 1:10 mit dem Bindungstest-Puffer verdünnt.

Im Bindungstest wurde als Puffer ein 50 mmol/l Tris-HCL, 5 mmol/l MgCl (pH 7,4) supplementiert mit 0,1 % (w/v) bovinem Serumalbumin, sowie als radioaktiver Ligand 1 nmol/l (³H)-2-D-Ala-Deltorphin II eingesetzt. Der Anteil an unspezifischer Bindung wurde in Anwesenheit von 10 mmol/l Naloxon bestimmt.

In weiteren Ansätzen wurden die erfindungsgemäßen Verbindungen in Konzentrationsreihen zugegeben und die Verdrängung des radioaktiven Liganden aus seiner spezifischen Bindung ermittelt. Die jeweiligen Dreifachansätze wurden über 90 Minuten bei 37°C inkubiert und anschließend zur Bestimmung des an das Membranhomogenat gebundenen radioaktiven Liganden mittels Filtration durch Glasfaserfilter (GF/B) geerntet. Die Radioaktivität der Glasfaserfilterscheiben wurde nach Zugabe von Szintillator im β-Counter gemessen.

Die Affinität der erfindungsgemäßen Verbindungen zum δ-Opiatrezeptor wurde als IC₅₀ nach dem Massenwirkungsgesetz mittels nichtlinearer Regression berechnet. Die Kᵢ-Werte in Tabelle 1 sind als Mittelwert ± Standardabweichungen von 3 voneinander unabhängigen Versuchen angegeben.

**Tabelle 1**

| Bsp. Nr. | δ-Opiatrezeptorbindung Kᵢ [nmol/l] |
|---|---|
| 1 | 10,2 ± 4,5 |
| 2b | 348 ± 65 |
| 2c | 137 ± 13 |
| 2d | 18,4 ± 1,6 |
| 2e | 8,5 ± 3 |
| 3 | 2,1 ± 0,13 |
| 5 | 68,2 ± 10,1 |
| 7 | 68,4 ± 8,4 |
| 8 | 393 ± 46 |
| 9a | 1,44 |
| 2g | 6,01 |
| 11b | 6,71 |
| 2h | 1,76 |

## Patentansprüche

1. Heterocyclische Benzocycloalkenderivate der allgemeinen Formel I 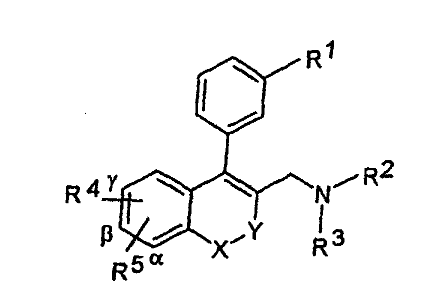 worin
R¹ OH, C₁₋₆-Alkoxy, O-(C₃₋₇)-Cycloalkyl;
R² C₁₋₆-Alkyl;
R³ C₁₋₆-Alkyl, -(CH₂)₁₋₂-Aryl, -(CH₂)₁₋₂-Heterocyclyl, -CH₂-CH=C(R⁶)₂, CH₂-(C₃₋₇)-Cycloalkyl;
R⁴ und R⁵ gleich oder verschieden voneinander H, Cl, F, CF₃, C₁₋₆-Alkyl, CON(R⁶R⁷), OH, C₁₋₆-Alkoxy, O-(C₃₋₇)-Cycloalkyl, α,β- oder β,γ -O-(CH₂)₁₋₂-O, -(CH₂)₀₋₂-Aryl, Heterocyclyl, -O-(CH₂)₀₋₂-Aryl, α,β- oder β,γ-Benzo unsubstituiert, mono- oder disubstituiert mit F, Cl, CF₃, OH, C₁₋₆-Alkyl, C₁₋₆-Alkoxy;
R⁶ H, C₁-C₆-Alkyl;
R⁷ H, C₁-C₆-Alkyl, -(CH₂)₀₋₂-Aryl, -CH₂-(C₃-C₇)-Cycloalkyl oder
R⁶ und R⁷ zusammengenommen (-CH₂-)₅₋₇ oder (-CH₂)₂-O-(CH₂)₂
X O, S, SO, SO₂ und
Y -(CH₂)₁₋₂-, -CH₂-C(CH₃)₂- oder -C(CH₃)₂-
bedeuten und deren pharmazeutisch verwendbare Salze.

2. Verbindungen gemäß Anspruch 1, **dadurch gekennzeichnet, daß** X O, S oder SO, Y -(CH₂)₁₋₂- und R¹ bis R⁷ die Bedeutung gemäß Anspruch 1 besitzen.

3. Verbindungen gemäß Anspruch 1, **dadurch gekennzeichnet, daß** X O, Y -(CH₂)₁₋₂- und R¹ bis R⁷ die Bedeutung gemaß Anspruch 1 besitzen.

4. Verbindungen gemäß Anspruch 1, **dadurch gekennzeichnet, daß** R⁴ und R⁵ gleich oder verschieden voneinander -O-(CH₂)₀₋₂-Aryl, -(CH₂)₀₋₂-Aryl oder Heterocyclyl, R¹ bis R³, R⁶, R⁷, X und Y die Bedeutung gemäß Anspruch 1 besitzen.

5. Verbindungen gemäß Anspruch 1, **dadurch gekennzeichnet, daß** R¹ C₁₋₆-Alkoxy oder -O-(C₃₋₇)-Cyclo-alkyl, R⁴ und R⁵ gleich oder verschieden voneinander -(CH₂)₀₋₂-Aryl oder Heterocyclyl, R², R³, R⁶, R⁷, X und Y die Bedeutung gemäß Anspruch 1 besitzen.

6. Verbindungen gemäß Anspruch 1, **dadurch gekennzeichnet, daß** R¹ OH oder C₁₋₆-Alkoxy bedeutet und R² bis R⁷, X und Y die Bedeutung des Anspruchs 1 besitzen.

7. Verbindungen gemäß Anspruch 1, **dadurch gekennzeichnet, daß** Y -(CH₂)₁₋₂-, R² C₁₋₆-Alkyl, X, R¹ und R³ bis R⁷, die Bedeutung des Anspruchs 1 besitzen.

8. Verbindungen gemaß Anspruch 1, **dadurch gekennzeichnet, daß** Y -(CH₂)₁₋₂-, R¹ OH oder C₁₋₆-Alkoxy, R² und R³ C₁₋₆-Alkyl, X und R⁴ bis R⁷ die Bedeutung gemäß der Definition des Anspruchs 1 besitzen.

9. Verbindungen gemäß Anspruch 8, **dadurch gekennzeichnet, daß** Y -(CH₂)₁₋₂-, R¹ OH, R² und R³ C₁₋₆-Alkyl, X O und R⁴ bis R⁷ die Bedeutung gemäß der Definition des Anspruchs 1 besitzen.

10. Verfahren zur Herstellung von heterocyclischen Benzocycloalkenderivaten der allgemeinen Formel I 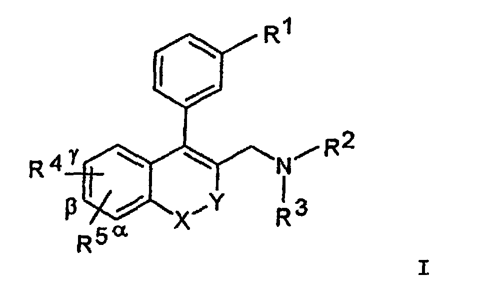 worin
R¹ bis R⁷, X und Y die Bedeutung gemäß Anspruch 1 besitzen, **dadurch gekennzeichnet, daß** ein tertiärer Alkohol der allgemeinen Formel II 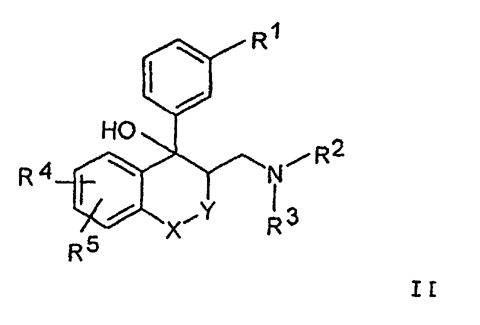 worin R¹ bis R⁷, X und Y dieselbe Bedeutung wie in Formel I haben, mit Sauren in einem Temperaturbereich von 0° - 100° C umgesetzt werden, wobei tertiäre Alkohole der allgemeinen Formel II dadurch erhalten werden, daß zunächst eine Mannich-Base der allgemeinen Formel III 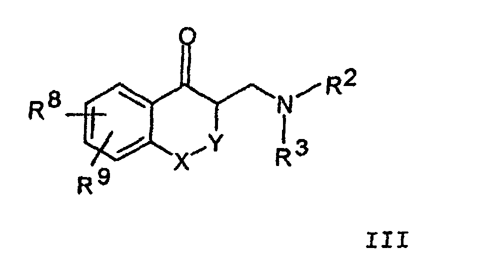 worin R², R³, X und Y die Bedeutung gemäß der allgemeinen Formel I besitzen und R⁸ wie R⁴ und R⁹ wie R⁵ definiert sind mit der Ausnahme, daß eine vorhandene Hydroxy-Funktion in geschützter Form als Silanyloxy- oder Benzyloxygruppe vorliegt, mit einer metallorganischen Verbindung der allgemeinen Formel IV, 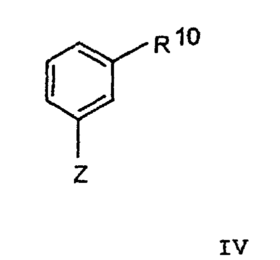 worin Z für MgCl, MgBr, MgI oder Li steht, und R¹⁰ die Bedeutung wie R¹ hat, mit der Ausnahme, daß eine gegebenenfalls vorhandene Hydroxy-Funktion in geschutzter Form als Benzyloxy- oder Silanyloxygruppe vorliegt, die zu einer Verbindung der allgemeinen Formel IIa 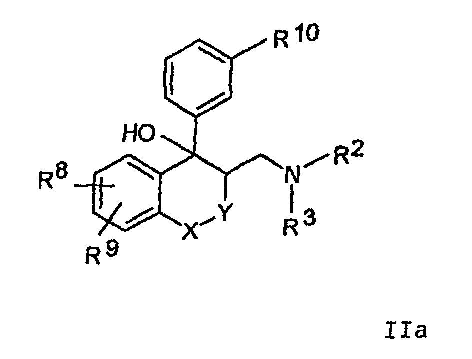 umgesetzt wird und diese dann in eine Verbindung der allgemeinen Formel II überführt, wird.

11. Verwendung der Verbindung der allgemeinen Formel I gemäß Anspruch 1 zur Herstellung eines Arzneimittels.

12. Verwendung nach Anspruch 11 **dadurch gekennzeichnet, daß** das Arzneimittel ein Analgetikum ist.

13. Arzneimittel, enthaltend wenigstens eine Verbindung der allgemeinen Formel I gemäß Anspruch 1 oder eines ihrer pharmazeutisch verwendbaren Salze.

## Claims

1. Heterocyclic benzocycloalkene derivatives of general formula I 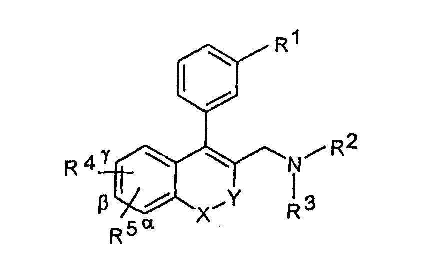 wherein
R¹ denotes OH, C₁₋₆ alkoxy or -O-(C₃₋₇) cycloalkyl;
R² denotes C₁₋₆ alkyl;
R³ denotes C₁₋₆ alkyl, -(CH₂)₁₋₂ aryl, -(CH₂)₁₋₂ heterocyclyl, -CH₂-CH=C(R⁶)₂, or -CH₂-(C₃₋₇) cycloalkyl;
R⁴ and R⁵, identically to or differently from each other, denote H, Cl, F, CF₃, C₁₋₆ alkyl, -CON(R⁶R⁷), OH, C₁₋₆ alkoxy, O-(C₃₋₇) cycloalkyl, α,β- or β,γ-O-(CH₂)₁₋₂-O-, -(CH₂)₀₋₂ aryl, heterocyclyl, -O-(CH₂)₀₋₂ aryl, or α,β- or β,γ-benzo which are unsubstituted, mono- or disubstituted with F, Cl, CF₃, OH, C₁₋₆ alkyl or C₁₋₆ alkoxy;
R⁶ denotes H or C₁-C₆ alkyl;
R⁷ denotes H, C₁-C₆ alkyl, -(CH₂)₀₋₂ aryl or -CH₂-(C₃-C₇) cycloalkyl, or
R⁶ and R⁷ taken together denote (-CH₂-)₅₋₇ or (-CH₂)₂-O-(CH₂)₂-,
X denotes O, S, SO or SO₂, and
Y denotes -(CH₂)₁₋₂-, -CH₂-C(CH₃)₂- or -C(CH₃)₂-,
or pharmaceutically acceptable salts thereof.

2. Compounds according to claim 1, **characterised in that** X denotes O, S or SO, Y denotes -(CH₂)₁₋₂- and R¹ to R⁷ have the meaning according to claim 1.

3. Compounds according to claim 1, **characterised in that** X denotes O, Y denotes -(CH₂)₁₋₂- and R¹ to R⁷ have the meaning according to claim 1.

4. Compounds according to claim 1, **characterised in that** R⁴ and R⁵, identically to or independently of each other, denote -O-(CH₂)₀₋₂-aryl, -(CH₂)₀₋₂ aryl or heterocyclyl, and R¹ to R³, R⁶, R⁷, X and Y have the meaning according to claim 1.

5. Compounds according to claim 1, **characterised in that** R¹ denotes C₁₋₆ alkoxy or O-(C₃₋₇) cycloalkyl, R⁴ and R⁵, identically to or differently from each other, denote -(CH₂)₀₋₂ aryl or heterocyclyl, and R², R³, R⁶, R⁷, X and Y have the meaning as in claim 1.

6. Compounds according to claim 1, **characterised in that** R¹ denotes OH or C₁₋₆ alkoxy and R² to R⁷, X and Y have the meaning as in claim 1.

7. Compounds according to claim 1, **characterised in that** Y denotes -(CH₂)₁₋₂-, R² denotes C₁₋₆ alkyl, and X, R¹ and R³ to R⁷ have the meaning as in claim 1.

8. Compounds according to claim 1, **characterised in that** Y denotes -(CH₂)₁₋₂-, R¹ denotes OH or C₁₋₆ alkoxy, R² and R³ denote C₁₋₆ alkyl, and X and R⁴ to R⁷ have the meaning according to the definition of claim 1.

9. Compounds according to claim 1, **characterised in that** Y denotes -(CH₂)₁₋₂-, R¹ denotes OH, R² and R³ denote C₁₋₆ alkyl, X denotes O and R⁴ to R⁷ have the meaning according to the definition of claim 1.

10. A method of producing heterocyclic benzocycloalkene derivatives of general formula I 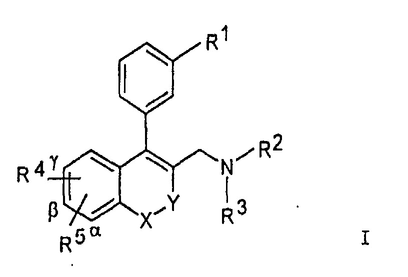 wherein
R¹ to R⁷, X and Y have the meaning according to claim 1, **characterised in that** a tertiary alcohol of general formula II 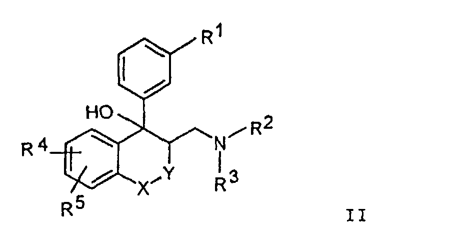 wherein R¹ to R⁷, X and Y have the same meaning as in formula I, is reacted with acids within a temperature range from 0°C - 100°C, wherein tertiary alcohols of general formula II are obtained firstly by the reaction of a Mannich base of general formula III 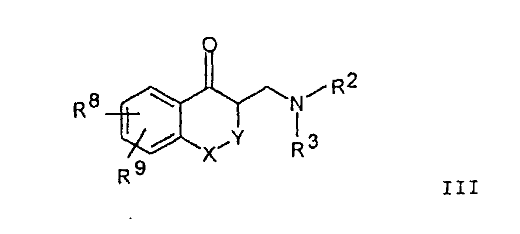 wherein R², R³, X and Y have the meaning according to general formula I, R⁸ is defined as is R⁴, and R⁹ is defined as is R⁵, with the exception that any hydroxy function which is exists is present in protected form as a benzyloxy or silanyloxy group, with an organometallic compound of formula IV 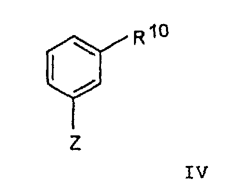 wherein Z represents MgCl, MgBr, MgI or Li, and R¹⁰ has the same meaning as R¹, with the exception that any hydroxy function which possibly exists is present in protected form as a benzyloxy or silanyloxy group, which is reacted to form a compound of general formula IIa 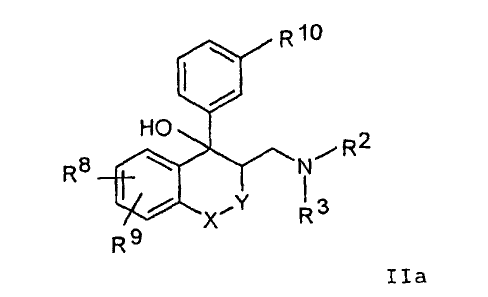 and the latter is then converted into a compound of general formula II.

11. A use of a compound of general formula I according to claim 1 for the production of a drug.

12. A use according to claim 11, **characterised in that** the drug is an analgesic.

13. A drug containing at least one compound of general formula I according to claim 1 or one of the pharmaceutically acceptable salts thereof.

## Revendications

1. Dérivés de benzocycloalcènes hétérocycliques de formule générale I 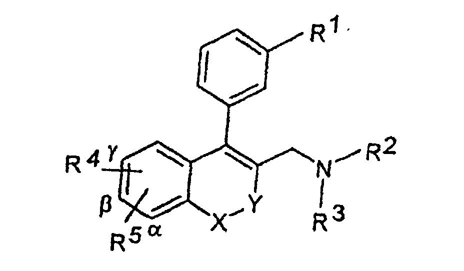 dans laquelle
R¹ représente un groupe OH, alkoxy en C₁ à C₆, O-(cycloalkyle en C₃ à C₇) ;
R² est un groupe alkyle en C₁ à C₆ ;
R³ est un groupe alkyle en C₁ à C₆, -(CH₂)₁₋₂-aryle, -(CH₂)₁₋₂-hétérocyclyle, -CH₂-CH=C(R⁶)₂, CH₂-(cycloalkyle en C₃ à C₇) ;
R⁴ et R⁵, identiques ou différents l'un de l'autre, représentent H, Cl, F, un groupe CF₃, alkyle en C₁ à C₆, CON(R⁶R⁷), OH, alkoxy en C₁ à C₆, O-(cycloalkyle en C₃ à C₇), α,β- ou β-γ-O-(CH₂)₁₋₂-O-, -(CH₂)₀₋₂-aryle, hétérocyclyle, -O-(CH₂)₀₋₂-aryle, α,β- ou β-γ-benzo non substitué, monosubstitué ou disubstitué avec F, Cl, CF₃, OH, alkyle en C₁ à C₆, alkoxy en C₁ à C₆ ;
R⁶ représente H ou un groupe alkyle en C₁ à C₆ ;
R⁷ représente H, un groupe alkyle en C₁ à C₆, -(CH₂)₀₋₂-aryle, -CH₂-(cycloalkyle en C₃ à C₇), ou bien
R⁶ et R⁷ forment conjointement un groupe (-CH₂-)₅₋₇ ou (-CH₂)₂-0-(CH₂)₂-,
X représente O, S, SO, SO₂ et
Y est un groupe -(CH₂)₁₋₂-, -CH₂-C(CH₃)₂- ou -C(CH₃)₂-
et leurs sels acceptables du point de vue pharmaceutique.

2. Composés suivant la revendication 1, **caractérisés en ce que** X représente O, S ou SO, Y est un groupe -(CH₂)₁₋₂- et R¹ à R⁷ ont la définition selon la revendication 1.

3. Composés suivant la revendication 1, **caractérisés en ce que** X représente O, Y est un groupe -(CH₂)₁₋₂- et R¹ à R⁷ ont la définition indiquée dans la revendication 1.

4. Composés suivant la revendication 1, **caractérisés en ce que** R⁴ et R⁵, identiques ou différents l'un de l'autre, représentent un groupe -O-(CH₂)₀₋₂-aryle, -(CH₂)₀₋₂-aryle ou hétérocyclyle, R¹ à R³, R⁶, R⁷, X et Y ont les définitions indiquées dans la revendication 1.

5. Composés suivant la revendication 1, **caractérisés en ce que** R¹ est un groupe alkoxy en C₁ à C₆ ou -O-(cycloalkyle en C₃ à C₇), R⁴ et R⁵ identiques ou différents l'un de l'autre représentent un groupe -(CH₂)₀₋₂-aryle ou hétérocyclyle, R², R³, R⁶, R⁷, X et Y ont les définitions indiquées dans la revendication 1.

6. Composés suivant la revendication 1, **caractérisés en ce que** R¹ est un groupe OH ou alkoxy en C₁ à C₆ et R² à R⁷, X et Y ont les définitions indiquées dans la revendication 1.

7. Composés suivant la revendication 1, **caractérisés en ce que** Y est un groupe -(CH₂)₁₋₂-, R² est un groupe alkyle en C₁ à C₆, X, R¹ et R³ à R⁷ ont les définitions indiquées dans la revendication 1.

8. Composés suivant la revendication 1, **caractérisés en ce que** Y est un groupe -(CH₂)₁₋₂-, R¹ est un groupe OH ou alkoxy en C₁ à C₆, R² et R³ sont des groupes alkyle en C₁ à C₆ et X et R⁴ à R⁷ ont les définitions indiquées dans la revendication 1.

9. Composés suivant la revendication 8, **caractérisés en ce que** Y est un groupe -(CH₂)₁₋₂-, R¹ est un groupe OH, R² et R³ sont des groupes alkyle en C₁ à C₆, X représente O et R⁴ à R⁷ ont les définitions indiquées dans la revendication 1.

10. Procédé de production de dérivés de benzocycloalcènes hétérocycliques de formule générale I 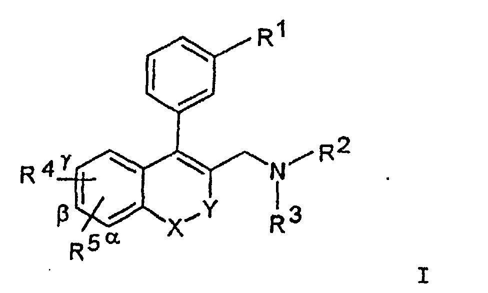 dans laquelle
R¹ à R⁷, X et Y sont tels que définis selon la revendication 1, **caractérisé en ce qu'**on fait réagir un alcool tertiaire de formule générale II 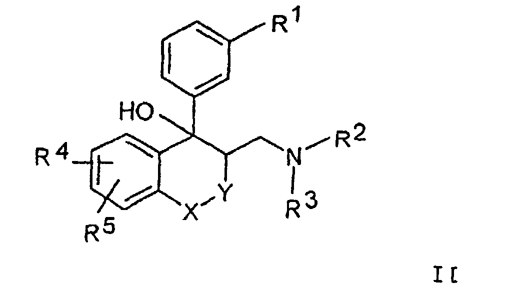 dans laquelle R¹ à R⁷, X et Y ont la même définition que dans la formule I, avec des acides dans une plage de température de 0° à 100°C, les alcools tertiaires de formule générale II étant obtenus par le fait qu'on fait réagir tout d'abord une base de Mannich de formule générale III 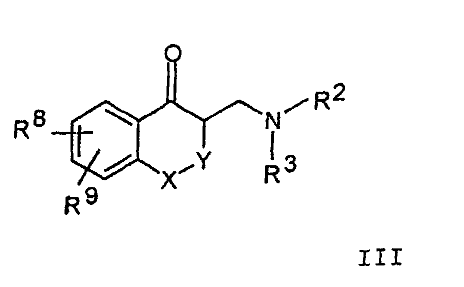 dans laquelle R², R³, X et Y ont la définition indiquée selon la formule générale I et R⁸ est défini comme R⁴, et R⁹ est défini comme R⁵, excepté qu'une fonction hydroxy présente existe sous la forme protégée comme groupe silanyloxy ou benzyloxy, avec un composé organométallique de formule générale IV 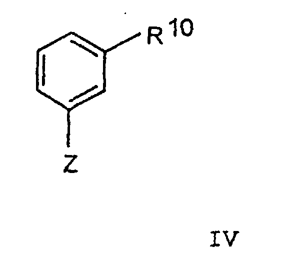 dans laquelle X représente MgCl, MgBr, MgI ou Li et R¹⁰ a la même définition que R¹, excepté qu'une fonction hydroxy éventuellement présente existe sous la forme protégée comme groupe benzyloxy ou silanyloxy, on transforme le produit en un composé de formule générale IIa 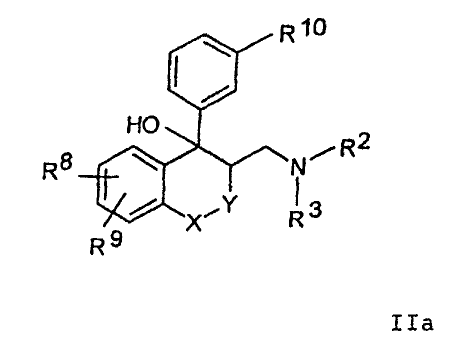 et on transforme ce composé en un composé de formule générale II.

11. Utilisation du composé de formule générale I suivant la revendication 1 pour la préparation d'un médicament.

12. Utilisation suivant la revendication 11, **caractérisée en ce que** le médicament est un analgésique.

13. Médicament contenant au moins un composé de formule générale I suivant la revendication 1 ou l'un de ses sels acceptables du point de vue pharmaceutique.
